# EUROPÄISCHE PATENTANMELDUNG

(11) **EP 4 813 199 A1**
(43) Veröffentlichungstag der Anmeldung: **30.09.2026**
(21) Anmeldenummer: 25166098.1
(22) Anmeldetag: 25.03.2025
(51) Int. Cl.: A01H 4/00, A01H 6/00, A01H 6/76

(54) **VERFAHREN ZUR HERSTELLUNG VON ZELLKULTIVIERTEN PFLANZENSAMEN, BOHNEN, KERNEN UND/ODER NÜSSEN**

(71) Anmelder: 10 X Innovation GmbH & Co. KG, 32427 Minden (DE)
(72) Erfinder: BLAESER, Andreas, 60327 Frankfurt am Main (DE); REINEL, Svenja, 64283 Darmstadt (DE); SCHOLPP, Sebastian, 64287 Darmstadt (DE); MAATZ, Robin, 63225 Langen (DE); SCHNELLBÄCHER, Kira, 64289 Darmstadt (DE); KORTE, René, 30161 Hannover (DE)
(74) Vertreter: Dantz, Jan Henning

(57) **Zusammenfassung**

Eine Schüttung umfassend eine Mehrzahl von Nüssen, Kerne, Pflanzensamen und/oder Bohnen, insbesondere Kaffeebohnen (107, 108), einer Pflanze. wobei jede der Nüsse, Kerne, Pflanzensamen und/oder Bohnen ein immobilisiertes pflanzeneigenes Zellmaterial aufweist, wobei die Gesamtheit des pflanzeneigenes Zellmaterials der Nüsse, Kerne, Pflanzensamen und/oder Bohnen Somaklone aufweist, die auf mehrere Nüsse, Kerne, Pflanzensamen und/oder Bohnen verteilt sind, sowie ein Verfahren zur Herstellung von biotechnologisch-hergestellten Nüssen, Kernen, Pflanzensamen und/oder Bohnen.

## Beschreibung

Die vorliegende Erfindung betrifft eine Schüttung aus Pflanzensamen, Bohnen, Kernen und/oder Nüssen und die Herstellung von zellkultivierten Pflanzensamen, Bohnen, Kernen und/oder Nüssen, genauer gesagt von biotechnologisch hergestellten Pflanzensamen, Kernen, Nüssen oder Bohnen umfassend immobilisiertes pflanzeneigenes kultiviertes Zellmaterial einer zum Pflanzensamen, zur Bohne, zum Kern und/oder zur Nuss korrespondierenden Pflanze.

Insbesondere betrifft die Erfindung ein Verfahren zur biotechnologischen Herstellung von Kaffeebohnen basierend auf zellkultiviertem Material.

Die Zellkultivierung und der damit verbundene Forschungsbereich der zellulären Landwirtschaft zur Bereitstellung von Lebensmitteln ist ein aktueller Forschungsschwerpunkt bei der Bekämpfung von Ursachen und Folgen des Klimawandels.

Alle bekannten Ansätze beruhen auf der Vermehrung von Zellkulturen unter Bereitstellung von hochkonzentrierten pulverförmigen und/oder granularen Materialien, die ggf. zu Pillen und dergleichen verpresst werden.

Der aktuelle Trend im Bereich der Kaffeezubereitung geht allerdings immer mehr in die Bereitstellung von Kaffeebohnen an den Endkunden zur Frischvermahlung und -verarbeitung zu koffeinhaltigen Heißgetränken.

Auch andere Produkte, wie Mandeln, Walnüsse und Haselnüsse, Pinienkerne, Chiasamen und dergleichen sind nicht in gemahlener Form erwünscht, sondern in ihrer ursprünglich geernteten Form.

Ausgehend von dieser Grundbetrachtung ist es die Aufgabe der vorliegenden Erfindung eine Schüttung einer Mehrzahl an Nüssen, Kernen, Pflanzensamen und/oder Bohnen bereitzustellen, welche biotechnologisch hergestellt sind und zugleich geschmacklich als bezüglich der Textur möglichst dem Originalprodukt entsprechen.

Die vorliegende Erfindung löst diese Aufgabe durch die Merkmale des Anspruchs 1.

Überdies ist es die Aufgabe der vorliegenden Erfindung ein Verfahren bereitzustellen, zur Herstellung von möglichst originalgetreuen Nüssen, Kernen, Pflanzensamen und/oder Bohnen.

Die Erfindung löst diese Aufgabe durch die Merkmale des Anspruchs 4.

Speziell für Kaffeebohnen liegt die Aufgabe der vorliegenden Erfindung im Bereitstellen eines Verfahrens, welches eine Einstellung des Aromaprofils von gerösteten Kaffeebohnen ermöglicht.

Die Erfindung löst die Aufgabe durch die Merkmale des Anspruchs 11.

Eine erfindungsgemäße Schüttung umfasst eine, vorzugsweise sortenreine, Mehrzahl von Nüssen, Kerne, Pflanzensamen und/oder Bohnen einer Pflanze, insbesondere Kaffeebohnen, wobei jede der Nüsse, Kerne, Pflanzensamen und/oder Bohnen ein immobilisiertes pflanzeneigenes Zellmaterial aufweist.

Allerdings werden diese Verfahren bislang noch nicht auf Zellkultivierungsverfahren angewandt. Hier wird das Ziel der Nachbildung einer originalgetreuen Frucht bzw. eines original Samens genutzt. Diese Frucht oder Samen können, analog wie das Original, nachreifen, da die biotechnologisch-hergestellte Frucht oder Samen in einer bevorzugten Ausführungsvariante ebenfalls noch lebendes Material enthalten kann.

Nach der Nachreifung kann das Material analog zur Verarbeitung von konventionell-hergestellten Kaffeebohnen getrocknet und geröstet werden.

Die Besonderheit der biotechnologisch in Rahmen eines Zellkultivierungsverfahrens hergestellten Nüssen, Kernen, Pflanzensamen und/oder Bohnen ist das Auftreten der herstellungsbedingten Somaklone, die auf mehrere Nüsse, Kerne, Pflanzensamen und/oder Bohnen verteilt sind. Beispielsweise können zumindest 50%, vorzugsweise zumindest 80% aller Nüsse, Kerne, Pflanzensamen oder Bohnen der Schüttung zueinander korrespondierende Somaklone sein. Durch das hohe Auftreten an Somaklonen ist der Ursprung der Herstellung durch Zellkultivierung nachweisbar.

Im Zuge der Zellkultivierung kann das gewünschte Aromaprofil des Kaffees über alle Bohnen einer Schüttung hinweg gezielt beeinflusst werden. Das Auftreten von Fehlgeschmacksnoten durch einzelne Bohnen ist bei dieser Form der Herstellung im Vergleich zum konventionellen Anbau deutlich verringert.

In einer ersten bevorzugten Ausführungsvariante weist das immobilisierte pflanzeneigene Zellmaterial eine Vielzahl von lebenden Zellen, insbesondere einen nachreifungsfähigen Kallus auf. Diese Variante entspricht beispielsweise geernteten Kaffeebohnen, welche auch im konventionellen Abbau noch Nachreifen. Durch die Möglichkeit der Nachreifung wird das Aromaprofil der Kaffeebohnen ausgebildet oder verstärkt.

In einer zweiten Ausführungsvariante weist die Schüttung geröstete Kaffeebohnen auf. Nach der Röstung sind typischerweise keine lebenden Zellen mehr vorhanden. Somit schützt die vorliegende Erfindung sowohl rohe Kaffeebohnen als auch das geröstete Endprodukt an den Endkunden.

Weiterhin erfindungsgemäß ist ein Verfahren zur Herstellung von einer Nuss, einem Kern, einem Pflanzensamen und/oder einer Bohne einer Pflanze, insbesondere zur Herstellung einer vorbeschriebenen erfindungsgemäßen Schüttung.

Besonders bevorzugt betrifft das Verfahren die Herstellung einer Kaffeebohne einer Kaffeepflanze.

Die Nuss, der Kern, der Pflanzensamen und/oder die Bohne, insbesondere die Kaffeebohne, umfasst ein immobilisiertes pflanzeneigenes Zellmaterial.

Das Verfahren weist zumindest die folgenden Schritte auf:
I Entnahme eines Explantats einer Pflanze, die eine Nuss, einen Kern, einen Pflanzensamen oder einer Bohne ausbildet;
   Das Explantat kann z.B. aus einer Frucht oder einem Samen entnommen werden oder auch aus einem anderen Pflanzenteil, wie z.B. dem Blatt, dem Stängel oder der Wurzel.
II Vermehrung des Zellmaterials des Explantats, vorzugsweise unter Züchtung eines Kallus aus dem Explantat;
   Ein weiterer Schritt des Verfahrens ist die Vermehrung des Zellmaterials des Explantats. Dies kann zunächst auf oder in einem ersten Nährmedium, z.B. einem Nährboden unter Ausbildung eines Kallus erfolgen. Sodann kann ein Teil des Kallus als eine Flüssigkultur in einem flüssigen Nährmedium kultiviert werden. Die Vermehrung umfasst daher vorzugsweise mehrere Teilschritte.
III Überführung eines Teils des vermehrten lebenden Zellmaterials in ein Hydrogel unter Ausbildung einer formbaren Masse umfassend das lebende Zellmaterial.
   Ein weiterer Schritt ist die Überführung des vermehrten Zellmaterials in ein Hydrogel. Es ist für eine gute Verarbeitbarkeit bevorzugt eine homogene Mischung des Zellmaterials mit dem Hydrogel bereitzustellen. Weiter bevorzugt kann um diese Mischung eine Verkapselung mit Hydrogel vorgenommen werden als Schutzmaßnahme.

Schließlich erfolgt der Schritt einer Formgebung der formbaren Masse umfassend das Hydrogel und das Zellmaterial zu einer Nuss, einem Kern, einem Pflanzensamen oder einer Bohne der Pflanze des Schritts I.. Die Formgebung erfolgt dabei unter derartigen Bedingungen, die ein Nachreifen bzw. eine zweite Kultivierung des in der Nuss, Kern, Pflanzensamen oder Bohne enthaltenen Zellmaterials möglich macht.

Aus Gründen der ernährungsphysiologischen Verträglichkeit ist es von Vorteil, wenn das Hydrogel ein pflanzenbasiertes Hydrogel ist, welches besonders bevorzugt ausschließlich aus pflanzlichen Inhaltsstoffen und Wasser besteht.

Das Hydrogel kann besonders bevorzugt Agarose, Alginat, Agar und/oder Pektin, insbesondere als gelbildende Komponente, umfassen oder daraus bestehen.

Alternativ oder zusätzlich können die Hydrogele auch proteinbasiert aus nichtpflanzlichen Ursprung gebildet sein. Beispielsweise können rekombinant-hergestellten Proteine zur Gelbildung genutzt werden.

Bevorzugte Beispiele sind vorzugsweise Chitosan oder rekombinantes Kollagen.

Die Formgebung kann vorteilhaft und in massenproduktionstauglicher Art und Weise durch ein Gießverfahren, vorzugsweise durch ein Spritzgussverfahrens, oder ein Druckverfahren, vorzugsweise ein Siebdruckverfahren oder ein 3D-Biodruckverfahren, erfolgen.

Es ist überdies von Vorteil, wenn das Verfahren zumindest eine Messung der hergestellten Kaffeebohne, insbesondere hinsichtlich der Konzentration eines einzelnen oder mehrerer Inhaltsstoffe umfasst und eine Anpassung der Vermehrungsbedingungen in Schritt II und/oder des Verhältnisses zwischen Zellmaterial und Hydrogel zur Ausbildung der formbaren Masse in Schritt III basierend auf dieser Messung erfolgt.

Dadurch kann gezielt die Ausbildung des Aromaprofils beeinflusst werden. Unter einem Aromaprofil versteht man ein sensorisches Profil umfassend Geruchsstoffe, Geschmacksstoffe, die Süße, den Salzgehalt, den Säuregehalt, das Mundgefühl, den Nachgeschmack und die Ausgewogenheit der Einzelkomponenten und deren Wechselwirkung. Weiterhin umfasst das Aromaprofil die biochemische Zusammensetzung umfassend den Gehalt an Koffein, Saccharose, Trigonellin, Chlorogensäure und/oder verschiedene Fettsäuren, einschließlich Riacylglycerine, Sterole und/oder Tocopherole. Die Konzentration einer oder mehrerer dieser Substanzen kann für die Zellkultur, die formbare Masse, das daraus biotechnologisch-hergestellte rohe Produkt oder das getrocknete und/oder geröstete Produkt bestimmt werden und zumindest die Schritte II und III des Verfahrens können basierend auf dieser Bestimmung eingestellt werden.

Wie bereits zuvor erörtert wurde, kann das Verfahren im Anschluss an die Formgebung eine Nachverarbeitung umfassen, wobei die Nachverarbeitung eine Nachreifung und/oder eine Trocknung und/oder eine Röstung umfasst.

Die Nachverarbeitung kann zudem eine Fermentation zur Einstellung bzw. Beeinflussung eines Aromaprofils umfassen. Eine Fermentation erfolgt auch bei konventionell angebauten Kaffeebohnen, allerdings vorrangig mit dem Ziel der Entschleimung. Diese ist bei den vorgenannten biotechnologisch-hergestellten Produkten nicht erforderlich.

Die Konzentration an einzelnen oder mehreren Inhaltsstoffen, insbesondere eines oder mehrerer Aromastoffe, der nachgereiften, getrockneten und gerösteten Kaffeebohnen kann vorteilhaft gemessen werden und es kann eine Einstellung der Prozessparameter zumindest der Schritte II und III anhand der ermittelten Messwerte erfolgt.

Nachfolgend wird die Erfindung anhand einer Ausführungsvariante zur Herstellung von Kaffeebohnen näher erläutert. Es zeigen:

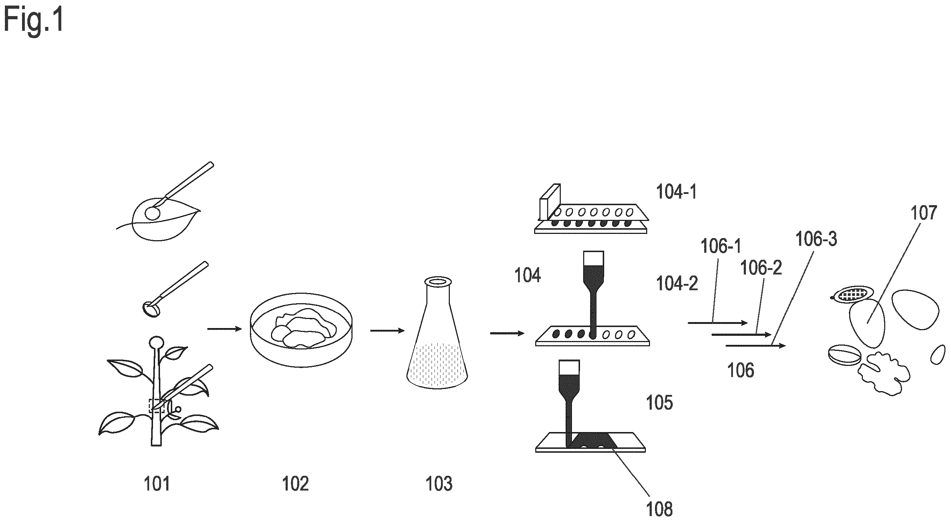

- Fig. 1: Schematische Darstellung eines Verfahrensablaufs bis zur Bereitstellung einer zellkultivierten Kaffeebohne.

Fig. 1 zeigt einen beispielhaften Verfahrensablauf eines erfindungsgemäßen Verfahrens. Wie aus Fig. 1 erkennbar, ist umfasst das Verfahren zumindest sechs Hauptschritte, die sich in mehrere Teilschritte unterteilen.

Der erste Schritt 101 umfasst die Entnahme eines Explantats aus einem pflanzlichen Organismus, welcher sich über die zu gewinnenden Pflanzensamen, Bohnen, Kerne und/oder Nüsse fortpflanzt. Konkret kann die Entnahme des Explantats von einer Kaffeepflanze 1 erfolgen.

Die Entnahme kann teilweise oder vollständig aus einem Pflanzenteil erfolgen, beispielsweise als Entnahme von Teilen eines Blattes, einer Wurzel, eines Stängels, eines Samens, einer Bohne, einer Nuss oder eines Kerns. Dabei werden Pflanzenzellen aus dem Blatt 2 der Kaffeepflanze 1 entnommen. Es ist auch die Entnahme von Endosperm einer Bohne möglich.

In einem zweiten Schritt 102 werden die Pflanzenzellen des Explantats in ein Nährmedium 3 platziert und unter Verwendung von gängigen Techniken in einen Kallus herangezogen.

Als Kallus wird ein Komplex von Pflanzenzellen bezeichnet, welcher aus dem vorgenannten Explantat, das vorher der lebenden Pflanze, z.B. einer Kaffeepflanze, entnommen wurde, entwickelt. Da die Zellen eines Kallus durch Mitose entstehen, sollten sie sich genetisch nicht unterscheiden.

Die Kultivierung des Kallus erfolgt ortsunabhängig von natürlichen Wachstumsgebieten der Pflanze. Dabei kann das Nährmedium eine Lösung oder auch ein Nährboden sein, welcher Saccharose, anorganische Salze, Vitamine, Proteine, und/oder Wachstumsregulatoren, sowie ggf. Hormone, aufweist. Als Wachstumsregulatoren können vorzugsweiseweise Auxine und/oder Zytokine verwendet werden, um das Zellwachstum zu gewährleisten.

Der Kallus besteht dabei aus Zellen, die in neue Gefäße übertragen werden, was zu einer beschleunigten Vermehrung der Zellmasse führt. Die Zellen sind als Somaklone ausgebildet, was allerdings nicht natürliche genetische Veränderungen ausschließt.

In einem dritten Schritt 103 erfolgt die Kultivierung in einer Flüssigkultur. Hierfür wird eine definierte Menge an Kallus in eine definierte Menge an einem Medium in eine Suspension umgewandelt. Das Suspensionsmedium kann einen voreingestellten Zuckergehalt und/oder einen definierten pH-Wert aufweisen. Das Nährmedium kann auf Grundlage etablierter Produkte hergestellt werden. Die Kultivierungsbedingungen können durch Schütteln, Rühren, durch Einstellung einer Temperatur der Suspension und/oder durch Einbringen einer Mindestmenge an Licht, Sauerstoff und/oder Luft eingestellt werden. Die Zellen können von einem Nährmedium in ein anderes, z.B. in zeitlich definierten Passagen, überführt werden oder die Inhaltsstoffe des Nährmediums können mehrmals überprüft und in Abhängigkeit von Zellwachstum eingestellt werden.

Der dritte Schritt kann auch eine Wachstumskontrolle umfassen. Dabei wird die Zusammensetzung, insbesondere die biochemische Zusammensetzung, der Zellkultursuspension mit geeigneten Methoden analysiert und mit der Zusammensetzung des Explantats verglichen. Bei dieser Art der Qualitätskontrolle kann überprüft werden, ob die Zusammensetzung der Zellkultur der Zusammensetzung des Explantats entspricht oder einer oder mehrere erwünschte Inhaltsstoffe gegenüber dem Explantat durch gezielt-eingestellte Kultivierungsbedingungen möglicherweise sogar erhöht sind.

In einem vierten Schritt 104 erfolgt eine Bereitstellung einer formbaren Masse, nachfolgend auch Biotinte genannt, zum Druck einer dreidimensionalen Struktur in Form einer Bohne, einer Nuss, eines Kerns und/oder eines Pflanzensamens. Der Begriff Biotinte bezieht sich im Kontext der nachfolgenden Beschreibung nicht ausschließlich auf Druckverfahren, sondern auch auf andere Verfahren der Formgebung, insbesondere auf Gießverfahren.

In einem ersten Teilschritt 104-1 erfolgt zunächst eine Isolierung und/oder Konzentrierung des kultivierten Zellmaterials der Flüssigkultur, z.B. durch Filtration. Die Filtration kann vorzugsweise durch eine Membranfiltration erfolgen. Es sind allerdings auch andere Filtrationstechniken möglich. Ebenfalls bevorzugt ist eine Filtration unter Normaldruck, unter geringem Unterdruck oberhalb von 100 mbar auf der Filtratseite oder Überdruck von weniger als 5 bar auf der Retentatseite. Dadurch werden die Zellen nicht zerstört.

Das konzentrierte Zellmaterial als Retentat kann vorteilhaft ebenfalls als Kallus vorliegen. Die Herstellung der Biotinte 104-2 erfolgt sodann durch Mischen des konzentrierten Zellmaterials mit einem geeigneten Hydrogel.

Bevorzugt umfasst das Hydrogel einen Gelbildner pflanzlichen Ursprungs.

Ein Hydrogel kann eine oder mehrere der folgenden Verbindungen als Gelbildner umfassen oder daraus bestehen: Pektin, Gelatin, Agarose, Gelatin-Methacrylol (GelMA), Kollagen, extrazellulares Matrixmaterial (EZM), Agar und/oder Alginat. Auch Chitosan kann als Basis eines Hydrogels genutzt werden

Weitere bevorzugte Verbindungen zur Bildung von Hydrogele oder als Inhaltsstoffe eines Hydrogels sind Chitosan, Cellulose, insbesondere Nanocellulose, Fibrin, Fibrinogen, Graphen, Hyaluronsäure und/oder Hydroxyapatit.

Besonders bevorzugte Gelbildner für die vorliegende Erfindung sind Pektin, Alginat und/oder Agarose. Agarose ist ein Polysaccharid, welches aus Seegras und anderen Wasserpflanzen gewonnen werden. Pektine sind Polysaccharide die aus Äpfeln, Zitruspflanzen und/oder Rüben gewonnen werden.

Innerhalb des Hydrogels kann das Zellmaterial bzw. ein daraus gebildeter Kallus nachreifen. Das im Hydrogel gebundene Wasser ermöglicht eine Versorgung des Zellmaterials.

Durch die Hydrogel-Matrix zur Verkapselung des Zellmaterials wird überdies eine Formgebung ermöglicht. Anhand von Vorversuchen konnte die Formgebung des Hydrogels für Pektin und Agarose zu einer Bohnenform bestätigt werden. Die Formgebung 105 bildet dabei den fünften Schritt des erfindungsgemäßen Verfahrens. Sie umfasst ein Gießen und/oder einen Geliermechanismus und einen anschließenden Trocknungsmechanismus. Alternativ kann die Formgebung auch durch Verpressen, analog zu einem Tablettierverfahren, erfolgen. Das Gießen kann z.B. in einem Spritzgussverfahren umgesetzt werden. Alternativ kann auch ein Sieb- oder Schablonendruckverfahren genutzt werden. Ein Spritzguss unter starker Materialerwärmung, wie beim Spritzguss von Thermoplasten, kann bei thermisch sehr resistentem Zellmaterial erfolgen, ist allerdings in den meisten Fällen aufgrund der Gefahr der Materialschädigung des lebenden Zellmaterials weniger bevorzugt. Daher empfiehlt sich bei der Formgebung eine Druck- und/oder Temperaturüberwachung.

Eine Alternative zum Gießen stellt, wie zuvor erwähnt, auch ein Biodruckprozess dar. Ein 3D-Biodruck kann durch eine Maschine realisiert werden, die eine Formgebung durch extrusionsbasierten Verfahren, lichtbasierten Verfahren und/oder tröpfchenbasierten Verfahren und/oder ein Siebdruckverfahren umfasst.

Ebenfalls bevorzugt ist ein Schablonendruckverfahren. Das Siebverfahren zeichnet sich dadurch aus, dass man ein Gewebe aufweist, das durch einen Rahmen gehalten wird.

Beim Schablonendruckverfahren wird eine Platte, z.B. aus Metall, genutzt, in welche Öffnungen, z.B. mittels Laser, eingebracht sind.

Beim Druck wird das Material durch die Platte oder das Gewebe in eine Form gebracht.

Einzelne Druckparameter, wie z.B. Abgabemenge pro Zeit, Mediumstemperatur und/oder Düsendruck können im Rahmen des Druckverfahrens, insbesondere des 3D-Biodruckverfahrens, insbesondere in Abhängigkeit vom verwendeten Material, z.B. von der Konzentration an Zellmaterial im Matrixmaterial, eingestellt werden.

Weitere einstellbare Druckparameter sind in Abhängigkeit von der gewählten Drucktechnik die Siebdicke, die Maschenweite, Öffnungsweite der Öffnungen der Platte, Materialstärke der Platte, Materialtyp der Platte, z.B. Metallsorte oder Kunststoff oder ähnliches, die Fadenstärke, die Rakelgeschwindigkeit, die Rakel- und/oder die Siebabsprunghöhe. Diese Parameter können ebenfalls auf die Zusammenstellung der Biotinte eingestellt werden

Besonders bevorzugt zur Formgebung einer Bohne, eines Kerns, einer Nuss, eines Pflanzensamens und insbesondere einer Kaffeebohne 108 ist ein Siebdruckverfahren. Allerdings können auch andere Verfahren wie Tiefdruck, Flexodruck, Inkjet-Druck, insbesondere basierend auf einer Abgabe der Biotinte aus Mikroventilen.

Ein weiterer Prozessparameter, welcher im Rahmen des erfindungsgemäßen Verfahrens einstellbar ist, ist das Substrat auf welchem die Formgebung erfolgt. Das Substrat kann funktionalisierte Oberflächen aufweisen. Die funktionalisierte Oberfläche weist eine definierte Oberflächenrauhigkeit und/oder einen auf das aufgebrachte Material eingestellten Benetzungsgrad auf. Die Oberfläche kann somit z.B. hydrophil oder hydrophob eingestellt sein. Die Funktionalisierung der Oberfläche kann durch physikalische Oberflächenbearbeitung, z.B. eine Plasmabehandlung, aufweisen oder alternativ durch eine auf einen Substratkorpus aufgebrachte Beschichtung erfolgen.

Eine bevorzugte Prozessgeschwindigkeit für die Formgebung beträgt mehr als 0,1 m/s, vorzugsweise 2,0 m/s. Im Fall eines Druckverfahrens entspricht die Prozessgeschwindigkeit der Vorschubgeschwindigkeit, welche vorzugsweise kontinuierlich erfolgt. Im Fall eines Gießverfahrens entspricht die Prozessgeschwindigkeit einer Zykluszeit.

Bevorzugt kann die Aushärtung, insbesondere beim 3D-Biodruck, durch Vernetzung erfolgen. Dies kann eine physikalische oder chemische Vernetzung umfassen. Die physikalische Vernetzung umfasst unter anderem thermische Vernetzungsmechanismen, Ionische Verbindungen und Verschlaufungen (z.B. durch Ausbildung von Wasserstoffbrücken). Die chemische Vernetzung umfasst die Vernetzung durch Ausbildung von kovalenten Bindungen, die z.B. nass-chemisch oder Licht-induziert erzeugt werden können. Durch das Aushärten wird dem Material eine mechanisch-stabile Form verliehen.

Ein der Maschine zugeordneter Extruder baut durch Einleiten der Matrixmaterials in sogenannte Formnester einer Formplatte Formen aus dem Hydrogel-Matrixmaterial auf. Die Hydrogelstruktur kann während der Formgebung und/oder danach durch Vernetzung, z.B. durch licht-, druck-, ionisch-bedingt und/oder wärmebedingte Nachvernetzung, aushärten.

Somit ermöglicht dieses Hydrogel-Material die Verkapselung der Pflanzenzellen bzw. des Kallus und die Ausformung zu einer Kaffeebohne. Die durch Formgebung bereitgestellte Bohne weist eine sehr harte Form auf.

In einem sechsten Schritt kann eine Nachverarbeitung 106 erfolgen.

Die Nachverarbeitung 106 kann mehrere Teilschritte umfassen.

Ein Teilschritt der Nachverarbeitung 106 kann z.B. eine zweite Kultivierung 106-1 umfassen. Dies kann unter kontrollierten Bedingungen, insbesondere zumindest durch Einstellung von Temperatur, Licht und Luftfeuchte, mittels eines Bioreaktors erfolgen. Durch die zweite Kultivierung 106-1 kann ein sortenindividueller Geschmack, im Fall von Kaffee ein sortenspezifisches Kaffeearoma, eingestellt werden.

Bei einer Bereitstellung von Kaffeebohnen nach dem konventionellen Verfahren gilt es eine Schleimschicht zu entfernen. Hierfür werden Kaffeebohnen in Fermentationsbehältern bei festgelegter Temperatur und Luftfeuchtigkeit für 16-36 Stunden fermentiert. Dieser langwierige Prozessschritt kann im vorliegenden Verfahren vorteilhaft entfallen. Alternativ ist es möglich eine optionale Fermentation, vorzugsweise eine Fermentation in weniger als 12h, zur Ausbildung eines Aromaprofils durchzuführen. Eine solche geschmacksbildende Fermentation kann durch eine Auswahl einer Hefekultur, Enzyme und/oder Bakterienkultur in Abhängigkeit von der Zusammensetzung der Biotinte erfolgen.

Alternativ oder zusätzlich kann eine weitere Trocknung 106-2 als weiterer Zusatzschritt erfolgen. Insbesondere kann eine Trocknung der nachgereiften Kaffeebohnen erfolgen. Die Trocknung kann bei einer bevorzugten Temperatur zwischen 35-45°C erfolgen. Es kann in einer bevorzugten Variante des Verfahrens eine Trocknung durch Widerstandsheizelemente, durch Infrarotheizung und/oder durch Umlufttrocknung erfolgen.

Alternativ oder zusätzlich kann ein weiterer Schritt der Nachverarbeitung eine Röstung 106-3 unmittelbar der in Schritt 105 bereitgestellten Bohnen, Nüsse, Kerne und/oder Pflanzensamen erfolgen oder bevorzugt im Fall der Bereitstellung von Kaffeebohnen der nachgereiften oder besonders bevorzugt getrockneten und nachgereiften Kaffeebohnen erfolgen. Die Röstung kann vorzugsweise bei einer geeigneten Temperatur bei z.B. 180-220°C, vorzugsweise 195-205 °C, erfolgen. Die Röstdauer kann vorzugsweise weniger als 20 min, vorzugsweise zwischen 6-10 min, erfolgen. Anschließend kann eine Luftkühlung durchgeführt werden.

Bei Kaffeebohnen stellen die gerösteten Bohnen 107 sodann das Endprodukt dar, welches verpackt und ggf. aromaversiegelt in den Handel gelangen kann.

Für die Kaffeebohnen können vom Endkunden dann verschiedene Zubereitungsarten gewählt werden, z.B. zum Direktverzehr, ummantelt mit Schokolade oder als Dekormaterial für Süßspeisen und Konfekt oder aber zur Vermahlung zu Kaffeepulver in einem Kaffeevollautomaten oder einer Kleinmühle z.B. für die Zubereitung in Siebträgermaschinen.

Diese Schritte sind allerdings nicht mehr Teil des Verfahrens. Das Endprodukt sind verpackte Kaffeebohne und die anderen grobkörnigen vorgenannte Produkte in Abgrenzung von pulverförmigem Material.

Die biotechnologisch-hergestellten harten Kaffeebohnen sind somit dabei unter anderem aus pflanzeneigenem Material einer Kaffeepflanze hergestellt, allerdings nicht als Frucht einer Pflanze nach Abschluss des gesamten Wachstumszyklus, sondern aus Teilen der Pflanze, welche auch deutlich vor der Ausbildung der Frucht unter natürlichen Bedingungen, gewonnen werden.

Es können allerdings auch Zellen der Frucht der jeweiligen Pflanze, also der Kaffeebohne, als Basis einer Zellkultur genommen werden. Dieses Zellmaterial wird allerdings durch die Kallusbildung vermehrt, so dass aus einer einzelnen Kaffeebohne eine Vielzahl biotechnologisch-herstellten Kaffeebohnen umfassend pflanzeneigenes Material hergestellt werden können.

Das erfindungsgemäße Verfahren betrifft somit das wachsende Feld der sogenannten zellulären Landwirtschaft, welche einen wichtigen Beitrag gegen die Ursachen und Folgen des globalen Klimawandels darstellt.

Die überwiegende Zahl der Verfahren in diesem Gebiet dient allerdings der Bereitstellung von pulverförmigen Produkten oder Zellmaterial. Eine Immobilisierung eines in zellulärer Landwirtschaft hergestellten Zellmaterials, welches nach der Immobilisierung noch nachreifen kann ist bislang nicht bekannt und stellt ein Novum dar.

Dabei kann das Produkt ausschließlich aus Materialien pflanzlichen Ursprungs bestehen.

### Bezugszeichen

- 101: Entnahme eines Explantats
- 102: Anzucht eines Kallus
- 103: Kultivierung in einer Flüssigkultur
- 104: Bereitstellung einer formbaren Masse
- 104-1: Isolierung und/oder Konzentrierung des kultivierten Zellmaterials
- 104-2: Mischen des konzentrierten Zellmaterials mit einem Hydrogel
- 105: Formgebung
- 106: Nachverarbeitung
- 106-1: zweite Kultivierung
- 106-2: Trocknung
- 106-3: Röstung
- 107: geröstete Kaffeebohnen
- 108: ausgeformte Kaffeebohnen

## Patentansprüche

1. Schüttung umfassend eine Mehrzahl von Nüssen, Kerne, Pflanzensamen und/oder Bohnen, insbesondere Kaffeebohnen (107, 108), einer Pflanze. wobei jede der Nüsse, Kerne, Pflanzensamen und/oder Bohnen ein immobilisiertes pflanzeneigenes Zellmaterial aufweist, **dadurch gekennzeichnet, dass** die Gesamtheit des pflanzeneigenes Zellmaterials der Nüsse, Kerne, Pflanzensamen und/oder Bohnen Somaklone aufweist, die auf mehrere Nüsse, Kerne, Pflanzensamen und/oder Bohnen verteilt sind.

2. Schüttung nach Anspruch 1, **dadurch gekennzeichnet, dass** das immobilisierte pflanzeneigene Zellmaterial eine Vielzahl von lebenden Zellen, insbesondere einen nachreifungsfähigen Kallus aufweist.

3. Schüttung nach Anspruch 1, **dadurch gekennzeichnet, dass** die Schüttung aus gerösteten Kaffeebohnen (107) besteht.

4. Verfahren zur Herstellung einer Nuss, einem Kern, einem Pflanzensamen und/oder einer Bohne, insbesondere einer Kaffeebohne (107, 108), einer Pflanze umfassend ein immobilisiertes pflanzeneigenes zellkultiviertes Zellmaterial, **gekennzeichnet durch die folgenden Schritte:**
I Entnahme eines Explantats (101) einer Pflanze, die eine Nuss, einen Kern, einen Pflanzensamen oder einer Bohne ausbildet;
II Vermehrung des Zellmaterials des Explantats, vorzugsweise unter Züchtung eines Kallus (102) aus dem Explantat;
III Überführung eines Teils des vermehrten lebenden Zellmaterials in ein Hydrogel unter Ausbildung einer formbaren Masse (104) umfassend das lebende Zellmaterial und
IV Formgebung (105) der formbaren Masse zu einer Nuss, einem Kern, einem Pflanzensamen oder einer Bohne der Pflanze des Schritts I.

5. Verfahren nach Anspruch 4, **dadurch gekennzeichnet, dass** die Vermehrung des Zellmaterials eine Züchtung eines Kallus (102) in einem Nährmedium und die Kultivierung des Kallus oder eines Teils des Kallus in einer Flüssigkultur (103) umfasst.

6. Verfahren nach einem der vorhergehenden Ansprüche, **dadurch gekennzeichnet, dass** das Hydrogel ein pflanzenbasiertes und/oder proteinbasiertes Hydrogel ist, welches besonders bevorzugt ausschließlich aus pflanzlichen Inhaltsstoffen und Wasser besteht.

7. Verfahren nach einem der vorhergehenden Ansprüche, **dadurch gekennzeichnet, dass** das Hydrogel Agarose, Agar, Alginat, Chitosan, Kollagen und/oder Pektin umfasst oder daraus besteht.

8. Verfahren nach einem der vorhergehenden Ansprüche, **dadurch gekennzeichnet, dass** die Formgebung (105) durch ein Gießverfahren, vorzugsweise eines Spritzgussverfahrens, oder ein Druckverfahren, vorzugsweise ein Siebdruckverfahren, ein Schablonendruckverfahren oder ein 3D-Biodruckverfahren, erfolgt.

9. Verfahren nach einem der vorhergehenden Ansprüche, **dadurch gekennzeichnet, dass** das Verfahren zumindest eine Messung der hergestellten Kaffeebohne (107, 108) und eine Anpassung der Vermehrungsbedingungen in Schritt II und/oder eine Anpassung des Verhältnisses zwischen Zellmaterial und Hydrogel zur Ausbildung der formbaren Masse in Schritt III umfasst.

10. Verfahren nach einem der vorhergehenden Ansprüche, **dadurch gekennzeichnet, dass** das Verfahren eine Nachverarbeitung (106) umfasst, wobei die Nachverarbeitung eine zweite Kultivierung (106-1), insbesondere eine Nachreifung, und/oder eine Trocknung und/oder eine Röstung umfasst.

11. Verfahren nach einem der vorhergehenden Ansprüche, **dadurch gekennzeichnet, dass** die zweite Kultivierung (106-1) eine Fermentation zur Einstellung eines Aromaprofils erfolgt.

12. Verfahren nach einem der vorhergehenden Ansprüche, **dadurch gekennzeichnet, dass** die Konzentration an Inhaltsstoffe, insbesondere an Aromastoffe, der nachgereiften, getrockneten und gerösteten Kaffeebohnen (107) ermittelt wird und dass eine Einstellung der Prozessparameter der Schritte II und III anhand der ermittelten Messwerte erfolgt.
